(19) Europäisches Patentamt
European Patent Office
Office européen des brevets

(11) **EP 3 738 511 B1**

(12) **EUROPÄISCHE PATENTSCHRIFT**

(45) Veröffentlichungstag und Bekanntmachung des Hinweises auf die Patenterteilung:
**26.04.2023** **Patentblatt 2023/17**

(21) Anmeldenummer: **20174922.3**

(22) Anmeldetag: **15.05.2020**

(51) Internationale Patentklassifikation (IPC):
**A61B 5/20** (2006.01)        **A61B 5/00** (2006.01)

(52) Gemeinsame Patentklassifikation (CPC):
**A61B 5/202; A61B 5/0075; A61B 2505/07**

(54) **NICHT-INVASIVE MESSVORRICHTUNG ZUR MESSUNG EINER FLÜSSIGKEITSEINLAGERUNG IN DER HARNBLASE EINES BENUTZERS**

NON-INVASIVE MEASURING DEVICE FOR MEASURING THE RETENTION OF LIQUID IN THE URINARY BLADDER OF A USER

DISPOSITIF DE MESURE NON INVASIF PERMETTANT DE MESURER UNE RETENTION LIQUIDIENNE DANS LA VESSIE D'UN PATIENT

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **16.05.2019   DE 102019207154**

(43) Veröffentlichungstag der Anmeldung:
**18.11.2020   Patentblatt 2020/47**

(73) Patentinhaber: **inContAlert GmbH**
**90619 Trautskirchen (DE)**

(72) Erfinder:
• **LOCKL, Jannik**
**90619 Trautskirchen (DE)**
• **ZÜRL, Tristan**
**95497 Goldkronach-Brandholz (DE)**

(74) Vertreter: **Hoffmann Eitle**
**Patent- und Rechtsanwälte PartmbB**
**Arabellastraße 30**
**81925 München (DE)**

(56) Entgegenhaltungen:
**WO-A1-2017/137350        WO-A1-2018/175100**
**US-A1- 2006 276 712**

**Beschreibung**

**Technisches Gebiet**

Die vorliegende Erfindung bezieht sich auf eine nicht-invasive Vorrichtung zur Messung einer Flüssigkeitseinlagerung in der Harnblase eines Benutzers und zur Ausgabe eines Messungsergebnisses.

**Hintergrund**

Eine nicht-invasive Messvorrichtung zur Messung einer Flüssigkeitseinlagerung kann z.B. Harninkontinenz betroffenen Personen helfen, Urineinlagerung zu erkennen. Alle Formen der Harninkontinenz haben gemeinsam, dass sie den Betroffenen ab einem gewissen Schweregrad der Störung zur Nutzung von aufsaugenden (Windeln, Einlagen) oder ableitenden (Katheter) Hilfsmitteln veranlassen und damit die Lebensqualität des Betroffenen beträchtlich einschränken können.

Personen mit einer neurogenen Blasenfunktionsstörung (z.B. Para- oder Tetraplegiker, Multiple Sklerose-, Morbus Parkinson-, Schlaganfalls-, Spina Bifida-Patienten, oder Patienten mit einer Neoblase) können sich aufgrund fehlenden Wissens über einen zu hohen Füllstand nicht eigenständig vor Harnrückstau schützen. Neben Inkontinenzerscheinungen (z.B. tröpfelnder Harnverlust oder unkontrollierte Entleerung der Blase) kann es jedoch auch zu einer Überfüllung der Blase bzw. dem vesikorenalen Reflux kommen. In diesen Fällen droht ein unphysiologischer Rücklauf des Harns in die Nierenbecken, was gesundheitsschädigend ist.

Nahinfrarotspektroskopie (NIRS) kann hierbei ein nichtinvasives Verfahren sein, das der Erkennung von Urineinlagerung durch optische Durchleuchtung von organischem Gewebe dient. Bei NIRS wird ein Lichtstrahl einer Infrarotstrahlungsquelle in das zu untersuchende Gewebe eingestrahlt, an einer anderen Stelle wird die Intensität der austretenden Strahlung gemessen und mit der des Ursprungssignals verglichen. Das Verhältnis der Signalamplituden gibt Aufschluss über die Extinktion (Schwächung) und Streuung des Lichtstrahls. Charakteristische Abhängigkeiten der Extinktionskoeffizienten der Wellenlänge werden durch variable Mengenanteile von Fett, Wasser oder Hämoglobin (je nach Sauerstoffsättigung) im Gewebe hervorgerufen, sodass die Möglichkeit besteht, Aussagen über die Konzentration dieser Stoffe bzw. Stoffgruppen im beleuchteten Bereich des Gewebes zu treffen.

Die Wasserkonzentration in menschlichem Gewebe kann Aufschluss über eine Vielzahl an Vorgängen im menschlichen Körper geben, wie z.B. den Muskelparametern bei Sportlern oder Informationen über das Gehirn. Molavi et al. (DOI: 10.1109/TBCAS.2013.2272013) beschreiben hierbei eine kabellose und tragbare Vorrichtung zur nichtinvasiven optischen Beobachtung des Blasenfüllzustandes mittels NIRS.

US 2006/0276712 A1 offenbart die Anwendung von NahinfrarotSpektroskopie (NIRS) in der Harnblasen-Urodynamik. Ein NIRS-Gerät wird dabei kalibriert, um die Intensität des Hintergrundfelds, die Hautfarbe und die Gewebedichte in einem iterativen Prozess zu berücksichtigen, der die Hinzufügung von Neutral-Dichte-Filtern mit optischer Dämpfung erfordern kann, um die physischen Parameter des Patienten zu berücksichtigen (z. B. Körpermasse, Hautpigmentierung, Hautdicke, Blasengröße, Detrusionsdicke, frühere Beckenoperationen, Adipositas, Fettpolster, Gewebewassergehalt). Nach der Kalibrierung liegt der Patient für eine beliebige Anzahl von Minuten in Rückenlage, die ausreicht, um eine stabile Basislinie für die Überwachung vor der Blasenentleerung zu erreichen. Bei einer stabilen Basislinie wird die NIRS-Überwachung in Intervallen von 1 bis 10 Hz fortgesetzt.

WO 2018/175100 A1 betrifft eine nicht-invasive optische Prüfsonde zur Ermittlung eines Blasenvolumens. Die Prüfsonde ist mit einer Vielzahl von LEDs und Fotodetektoren ausgestattet, wobei das Ein- und Ausschalten der LEDs von einer Elektroeinheit so geregelt wird, dass Messungen mit einer Abtastfrequenz von 1 Hz durchgeführt werden können. Wird einer der Fotodetektoren übersteuert, wird der LED-Strom reduziert, bis keine Übersteuerung stattfindet.

**Zusammenfassung der Erfindung**

**Technisches Problem**

Es ist jedoch noch nicht möglich, den Füllstand der Harnblase mit einer ausreichenden Genauigkeit zu bestimmen. Insbesondere ist in der herkömmlichen Technologie das Anregungssignal der Lichtquelle bzw. eine aus der Lichtquelle austretende Strahlung nicht auf die spezielle Anatomie und Körperzusammensetzung eines individuellen Benutzers (von Harninkontinenz betroffene Person, Querschnittspatient usw.) angepasst.

Der vorliegenden Erfindung liegt somit die Aufgabe zugrunde, das oben angeführte technische Problem zu lösen.

**Lösung des technischen Problems**

Der Gegenstand des unabhängigen Anspruchs 1 löst das oben angeführte technische Problem, während die abhängigen Ansprüche weitere bevorzugte Ausführungsformen verkörpern. Insbesondere wird hierbei ein an den Benutzer angepasstes Anregungssignal, das die Lichtausstrahlung steuert, eingesetzt, womit bspw. Frequenz, Amplitude und Pulsbreite des Anregungssignals an die Anatomie des Benutzers angepasst sind.

Somit ist es möglich, den Füllstand der Harnblase mit einem tragbaren Gerät präzise und kontinuierlich zu bestimmen bzw. zu überwachen. Somit kann ein Messergebnis mit einem für den Benutzer oder Patienten angepassten Grenzwert für die maximal noch zurückhaltbare Harnmenge verglichen und bei Überschreiten dieser kalibrierbaren Grenze eine Ausgabe ausgegeben werden. Die Ausgabe ist z.B. das Bereitstellen bzw. Ausgeben eines Messungsergebnisses für weitere Verarbeitung oder eine direkte Ausgabe eines Hinweissignals an den Patienten und/oder einen anderen, wie z.B. einen Arzt oder Betreuungspersonal. Der Benutzer kann dann vor der ungewollten Miktion eine Toilette aufsuchen oder dazu veranlasst werden. Ebenso kann die Ausgabe das Messungsergebnis oder eine Information bereitstellen und ggf. ein Alarm über das Erreichen eines Füllstands, welcher einen Rückstau oder Rücklauf in die Nierenregion verursachen könnte, ausgeben. Mit dieser Vorgehensweise lassen sich sonst notwendige Hilfsmittel, wie etwa der Bedarf zur Behandlung des Flüssigkeitsverlusts mit Windeln und zur Vorbeugung des Rückstaus mit Kathetern, einsparen, da das Wissen über den Füllstand der Harnblase vermittelt wird und somit präventive Maßnahmen ergriffen werden können, wodurch die Lebensqualität des Benutzers wieder deutlich erhöht werden kann.

**Kurzbeschreibung der Zeichnungen**

**FIG. 1** zeigt eine nicht-invasive Messvorrichtung zur Messung einer Flüssigkeitseinlagerung nach einer Ausführungsform der vorliegenden Erfindung.

**FIG. 2** zeigt einen Betrieb der nicht-invasiven Messvorrichtung zur Messung einer Flüssigkeitseinlagerung.

**FIG. 3** zeigt die Anordnung der nicht-invasiven Messvorrichtung zur Messung einer Flüssigkeitseinlagerung.

**FIG. 4** zeigt einen charakteristischen Verlauf der Extinktionskoeffizienten verschiedener Gewebearten über verschiedene Wellenlängen.

**FIG. 5** zeigt eine beispielhafte schematische Darstellung eines variablen Widerstandes.

**FIG. 6** zeigt eine beispielhafte schematische Darstellung einer Transimpedanz-Verstärkerschaltung.

**FIG. 7** zeigt eine beispielhafte schematische Darstellung einer Schaltung zum Betrieb von Leuchtdioden der Lichtquelle.

**FIG. 8** zeigt ein beispielhaftes emittiertes Rechtecksignal (Anregungssignal) mit Frequenz 1 kHz für eine Messung mit Messdauer T = 10 ms.

**FIG. 9** zeigt detektierte Signale nach Verstärkung und Bereinigung des Mittelwerts (durchgezogene Linie) und das zugrunde liegende Anregungssignal (gepunktet) aus zwei beispielhaften Messungen.

**Fig. 10** zeigt eine Autokorrelation eines mittelwertfreien Signals in verschiedenen Zeitausschnitten.

**Fig. 11** zeigt eine Kreuzkorrelation des mittelwertfreien Signals für verschiedene zeitliche Verschiebungen.

**Detaillierte Beschreibung**

Im Folgenden wird die vorliegende Erfindung unter Bezug auf die Zeichnungen detailliert beschrieben.

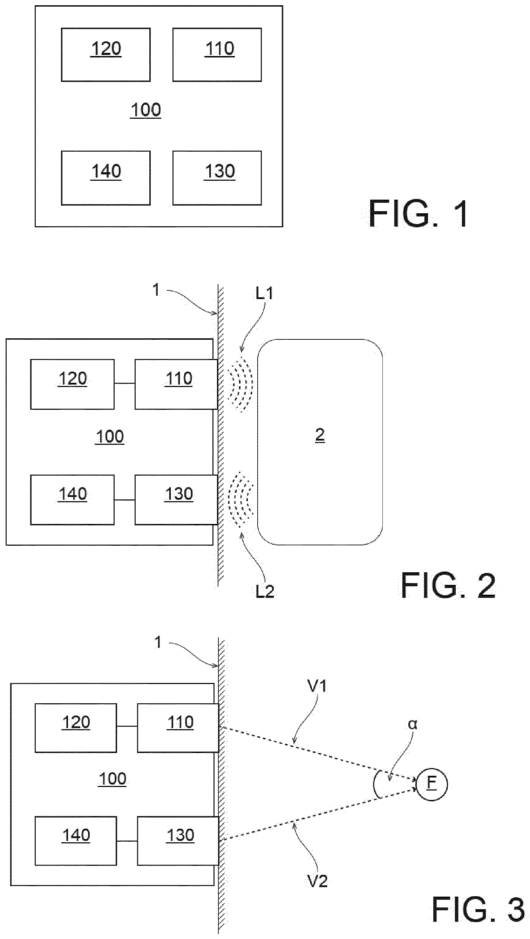

**FIG. 1** zeigt eine nicht-invasive Messvorrichtung 100 zur Messung einer Flüssigkeitseinlagerung nach einer Ausführungsform. Diese nicht-invasive Messvorrichtung 100 umfasst eine Lichtquelle 110, eine Steuereinheit 120, eine Messeinheit 130 und eine Auswerteeinheit 140. Der Betrieb dieser nicht-invasiven Messvorrichtung 100 wird beispielhaft in **FIG. 2** gezeigt. Hier liegt sie auf dem Körper 1 (bzw. der Körperoberfläche) eines Benutzers auf, um die Flüssigkeitseinlagerung in z.B. der (schematisch dargestellten) Harnblase 2 zu messen bzw. zu bestimmen. Die Lichtquelle 110, die Steuereinheit 120, die Messeinheit 130 und die Auswerteeinheit 140 werden im Folgenden detailliert beschrieben.

Die Lichtquelle 110 gemäß **FIG. 1** ist so ausgebildet, dass sie auf dem Körper bzw. der Körperoberfläche 1 des Benutzers anliegt und ein Licht L1, z.B. einen Lichtstrahl, ein Lichtbündel, oder dergleichen in den Benutzer einstrahlt. In diesem Fall ist der Benutzer eine Person, dessen Flüssigkeitseinlagerung gemessen werden soll, wobei in einem konkreteren Fall, die Flüssigkeitseinlagerung und insbesondere das Ausmaß bzw. die Menge der Flüssigkeitseinlagerung in der Harnblase 2 gemessen/bestimmt werden soll. Dies ist z.B. bei Inkontinenzbetroffenen, Querschnittspatienten und/oder Patienten, bei denen eine Störung eines harnbildenden oder ableitenden Organs vorliegt, vorteilhaft, da für sie gesundheitsschädigende Zustände vermieden, ein gewisser Anteil der sonst notwendigen Hilfsmittel eingespart und die Lebensqualität und Gesundheit erhöht wird.

Zur Erzeugung eines Lichtsignals, welches menschliches Gewebe ausreichend tief durchdringen kann, aber ebenfalls sehr sensitiv auf Änderung desselben reagiert, können prinzipiell beliebige Strahlungs- bzw. Lichtquellen, die in einem optischen Fenster (Wellenlängenbereich, bei denen eine geringe Schwächung der Strahlung im organischen Gewebe auftritt) emittieren, eingesetzt werden. Um einen komfortablen und gefahrlosen Einsatz in einer tragbaren, für den persönlichen Gebrauch der Benutzer/Patienten geeigneten Messvorrichtung zu ermöglichen, sind allerdings weitere Anforderungen zu erfüllen: schmales Emissionsspektrum mit einem Maximum, mechanische Robustheit, chemische Robustheit bei Kontakt mit menschlicher Haut bzw. deren Absonderungen, Gefahrlosigkeit bei Augenkontakt mit der emittierten Strahlung, Gefahrlosigkeit bei Hautkontakt mit der Strahlungsquelle, hoher Wirkungsgrad, niedrige Bauteilkosten.

Die Lichtquelle kann hier eine Nahinfrarotlichtquelle (NIR) sein, die eine oder mehrere Leuchtdioden aufweist und Licht in einem Wellenlängenbereich von 600 nm bis 1200 nm, bevorzugt zwischen 600 nm bis 1000 nm ausstrahlt, insbesondere vier Leuchtdioden für Licht im Bereich zwischen 830 nm und 950 nm. Beispiel für eine derartige Lichtquelle sind Laser und Leuchtdioden (LEDs). Vorzugsweise werden vier Leuchtdioden mit unterschiedlichen Wellenlängen, z.B. 660 nm, 830 nm, 940 nm und 950 nm oder z.B. 670 nm, 830 nm, 1020 nm und 1050 nm eingesetzt. Alternativ können auch 6 LEDs verwendet werden, z.B. mit den Wellenlängen 670 nm, 775 nm, 855 nm, 880 nm, 1020 nm, 1050 nm.

Der Einsatz von Lasern kann bevorzugt sein. Hervorzuheben sind hier Dioden-Laser, welche dank ihres kompakten Aufbaus auch in einer tragbaren Vorrichtung einfach untergebracht werden können. Die Breite des Emissionsspektrums ist bei Dioden-Lasern extrem gering, sodass durch den Einsatz verschiedener Typen sehr präzise definierte Punkte im Spektrum angeregt werden können.

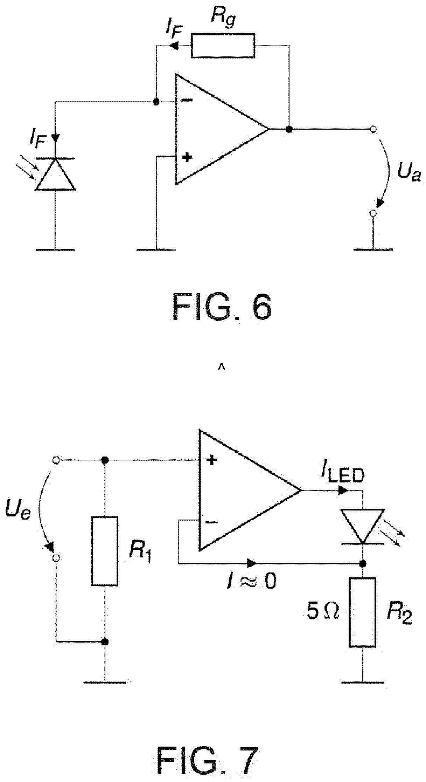

Leuchtdioden (LEDs) weisen ebenfalls ein relativ schmales Emissionsspektrum auf (etwa bis zu einer Halbwertsbreite von 30 nm). LEDs in mechanisch robusten und chemisch widerstandsfähigen bzw. gesundheitlich unbedenklichen Kunststoffgehäusen sind kostengünstig verfügbar. Weiterhin ist das Risiko einer Augenschädigung bei den geringen, hier verwendeten Leistungen nicht gegeben, da die von LEDs emittierte Strahlung inkohärent und nicht monochromatisch ist und sich somit nicht - wie z.B. bei einem Laser - auf sehr kleine Flächen fokussieren lässt. Der Wirkungsgrad von LEDs ist ausreichend hoch, sodass eine merkliche Erhitzung der Module im Betrieb nicht zu erwarten ist. Mit z.B. der in **Fig. 7** gezeigten Schaltung kann einer Leuchtdiode der Lichtquelle 110 ein LED-Strom $I_{LED}$ basierend auf einem im Folgenden erläuterten Anregungssignal bzw. -spannung $U_e$ zugeführt werden. In dem in **Fig. 7** gezeigtem Beispiel dient Widerstand $R_1$ als Pull-Down-Widerstand und $R_2$ als Messwiderstand zur Rückführung der LED-Stromstärke.

Die Steuereinheit 120 gemäß **FIG. 1** ist so ausgebildet, dass sie ein an den Benutzer angepasstes Anregungssignal, das die Lichtausstrahlung der Lichtquelle 110 steuert, an die Lichtquelle 110 ausgibt. Das Anregungssignal kann in diesem Fall ein Rechteckstromsignal mit einer Frequenz zwischen 500 Hz und 5 kHz, dessen Amplitude (Stromstärke) zwischen bspw. 20 mA und 1,5 A entsprechend der anzusteuernden Lichtquelle 110 einstellbar ist. Das Anregungssignal kann insbesondere an den Benutzer durch eine vor der eigentlichen Messung der Flüssigkeitseinlagerung durchgeführten Probemessung mit Testpulsen (die später genauer beschrieben wird) angepasst werden. Bei dieser Anpassung können die Werte der Frequenz und/oder Stromstärke durch Testpulse (der Probemessung) bestimmt werden, sodass eine optimale Lichtintensität von der im Folgenden beschriebenen Messeinheit 130 detektiert werden kann. Das Anregungssignal der Lichtquelle bzw. eine entsprechend aus der Lichtquelle austretende Strahlung kann somit an die spezielle Anatomie und/oder Körperzusammensetzung eines individuellen Benutzers angepasst werden, sodass das (im Folgenden erläuterte) Messsignal weder zu gering ist, wodurch Ungenauigkeiten in der Bestimmung des Ausmaßes der Flüssigkeitseinlagerungen vermieden werden können, noch zu groß ist, wodurch eine zu große thermische Belastung des Benutzers und/oder Übersteuerung der Messeinheit 130 vermieden wird.

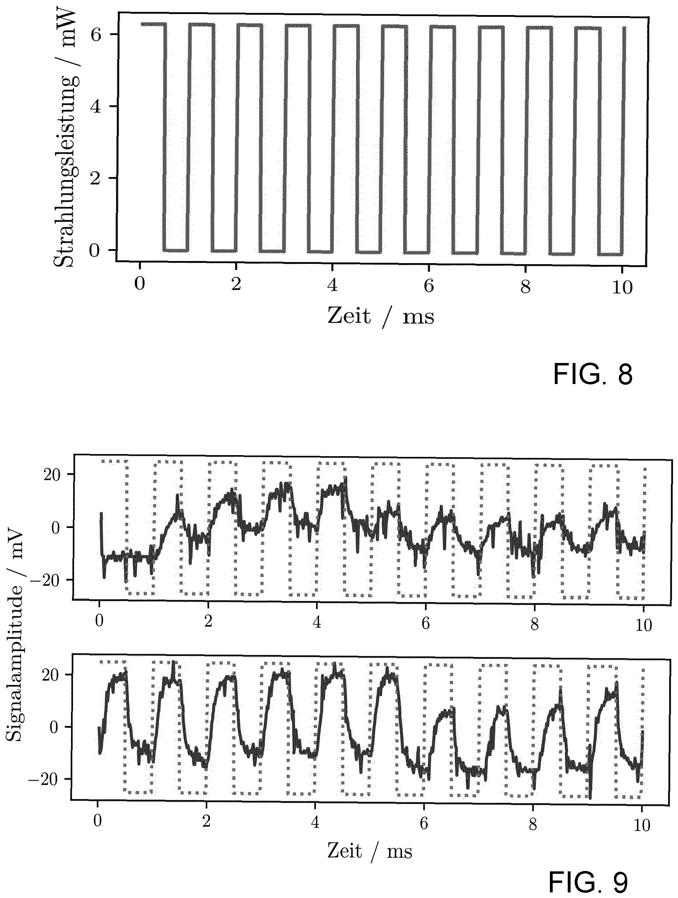

**Fig. 8** zeigt das Beispiel eines emittierten Rechtecksignals (Anregungssignal), dessen Amplitude (Strahlungsleistung in mW) mit einer Auflösung von z.B. 16 Bit, also in $2^{16} - 1 = 65535$ (siehe im Folgenden) verschiedenen Schritten eingestellt werden kann. Die Frequenz des Rechtecksignals kann in Abhängigkeit des Detektors und/oder an einen Benutzer individuell angepasst werden. Bevorzugt wird eine möglichst hohe Frequenz, um die gesamte Messzeit gering zu halten, während gleichzeitig auf die Bandbreite des Detektors Rücksicht genommen wird. Die Bandbreite des Detektors (OPT101) liegt bspw. bei $B_{DET}$ = 2.5 kHz. Es wird also bevorzugt eine Frequenz gewählt, die geringer als die halbe Bandbreite ist, um sicher zu gehen, dass der Detektor für Signale mit der Anregungsfrequenz empfindlich ist. Bspw. kann eine Frequenz $f_{EM}$ = 1 kHz gewählt werden. Für eine Messung wird das Anregungssignal dann für eine Messzeit von T = 10 ms, also einem 10 Perioden entsprechenden Intervall ausgegeben.

Zur Realisierung des an den Benutzer angepassten Anregungssignals kann die Steuereinheit 120 bspw. eine Treiberschaltung aufweisen, welche es ermöglicht, z.B. über ein Schieberegister die Stärke des durch die Lichtquelle 110 (z.B. Leuchtdioden) fließenden Stromes einzustellen. Unabhängig davon kann die Treiberschaltung aktiviert und deaktiviert werden, um Energie zu sparen, während diese nicht benötigt wird. Zusätzlich existiert die Möglichkeit, der Treiberschaltung ein Rechtecksignal zu übergeben, welches zu einer gepulsten Ansteuerung der Lichtquelle 110 (bzw. der Leuchtdioden als Infrarotlichtquellen) führt. Die Treiberschaltung ist bevorzugt eine Softwarekonfigurierbare Treiberschaltung, die ein PWM-Signal mit einstellbarer Stromamplitude ausgeben kann, wie im Folgenden beispielhaft erläutert wird. Somit kann ein an den Benutzer angepasstes Anregungssignal, das die Lichtausstrahlung steuert, erzeugt werden, wobei bspw. Frequenz, Amplitude und Pulsbreite des Anregungssignals an die Anatomie des Benutzers angepasst sind.

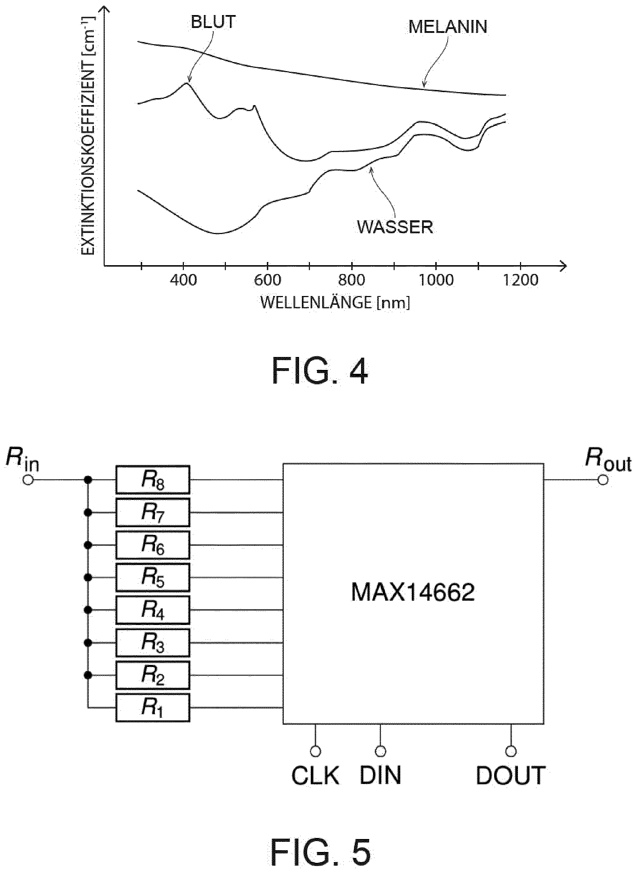

Eine beispielhafte Ausbildung der Treiberschaltung wird im Folgenden erläutert. Grundlage der Treiberschaltung ist ein integrierter Schaltkreis, z.B. der Schaltkreis STCS1A des Herstellers STMicroelectronics, der folgende Funktionen aufweist: maximal getriebener Strom ist 1,5A, Dimmungsmechanismus über PWM-Eingangspin, Shutdown-Pin, einstellbare Stromstärke über extern beschalteten Widerstand. Um die Amplitude des Stromes im Feld bzw. beim Einsatz der Messvorrichtung 100 einstellbar zu gestalten, kann ein Widerstandsnetzwerk aufgebaut werden, welches in der Lage ist, zwischen beiden Anschlüssen für den Stromeinstellungswiderstand einen per Software verstellbaren elektrischen Widerstand zu generieren. Dieses Widerstandsnetzwerk besteht z.B. aus 8 bis 16 parallel geschalteten Widerständen, welche über einen Analogschalter, z.B. des Typs MAX14662, einzeln zu- und abschaltbar sind, wie in **FIG. 5** gezeigt.

In **FIG. 5** ist die Struktur des Netzwerks vereinfacht dargestellt; eine tatsächlich realisierte Schaltung besteht aus einer Parallelschaltung von zwei hier dargestellten Schaltungen (die Eingänge $R_{in}$ und die Ausgänge $R_{out}$ werden jeweils miteinander verbunden) mit einem 10-Ohm-Widerstand (zur Einstellung eines Standardwerts). Die integrierten Schieberegister zur Konfiguration der Schalter können seriell miteinander verbunden werden, sodass eine sog. Daisy-Chain-Struktur entsteht. Hierfür wird der Dateneingang DIN des nachgeschalteten Schieberegisters mit dem Datenausgang DOUT des in der Kette vorher liegenden Bausteins verbunden, sodass effektiv ein Schieberegister mit doppelter Länge entsteht. Dies ermöglicht die Ansteuerung über ein Taktsignal sowie ein einzelnes Datensignal, während die Ansteuerungszeit für eine vollständige Erneuerung des Speicherinhaltes der gesamten Kette proportional zur Anzahl ihrer Glieder ist. Die absolute Ansteuerzeit bleibt hier gering, weswegen kein Nachteil durch die Daisy-Chain entsteht.

Die Messeinheit 130 gemäß **FIG. 1** ist so ausgebildet, dass sie auch auf dem Körper bzw. der Körperoberfläche 1 des Benutzers anliegt, dass sie das von der Lichtquelle 110 ausgestrahlte und von der Harnblase 2 zurückgeworfene Licht L2 erfasst und, dass sie ein dem erfassten Licht L2 entsprechendes Messsignal ausgibt. Das von der Lichtquelle 110 ausgegebene bzw. ausgestrahlte Licht tritt hier in den Körper des Benutzers 1 ein, wird entsprechend der Menge bzw. dem Ausmaß der in der Harnblase angesammelten Flüssigkeit absorbiert, und unterliegt auch einer Reflektion im Bereich der Harnblase, sodass das Licht L2 auf die Messeinheit 130 zurückgeworfen wird. Es ist hier ferner zu beachten, dass das Licht (L1 und L2) auf dem Weg durch den Körper ferner einer Streuung und/oder Extinktion (bzw. Schwächung, Absorption) unterliegt, die insbesondere von der speziellen Anatomie und/oder Körperzusammensetzung eines individuellen Benutzers abhängt. Eine starke Inhomogenität organischen Gewebes, die sich für jeden Nutzer unterscheiden kann, sorgt dabei für eine hochkomplexe Verkopplung aus Streuungs- und Extinktionseffekten auf dem Pfad des Lichts zwischen Strahlungsquelle (Lichtquelle 110) und Strahlungsdetektor (Messeinheit 130). Gerade bei tiefer liegenden zu überwachenden Organen ergibt sich somit eine Vielzahl an Störfaktoren, die das Messergebnis verfälschen bzw. überlagern können. Damit hängt das in der Messeinheit 130 erfasste Lichtsignal auch von den individuellen Besonderheiten des Benutzers ab und kann somit zu Messungenauigkeiten führen. Zum Ausgeben eines entsprechenden Messsignals kann in der Messeinheit 130 bspw. ein analoges Signal einer Fotodiode über eine Zwischen- oder Verstärkerschaltung an einen Analog-Digital-Umsetzer (ADU) geleitet werden, der das analoge Signal in ein zur Verarbeitung konvertiertes digitales Signal, bzw. ein digitales Messsignal umsetzt. Die Fotodiode kann in Verbindung mit entsprechender Beschaltung zu einem Infrarot-Detektor ausgebaut werden, welcher dann auf Basis von Infrarotbestrahlung ein der Infrarotintensität entsprechendes Signal (Spannungs- oder Stromsignal) ausgibt. Dieses kann dann bspw. von einem ADU abgetastet werden.

Zur vollständigen Konfiguration des Widerstandsnetzwerks sind gemäß diesem Beispiel der Treiberschaltung 16 Bits notwendig, wobei jeder Widerstand einzeln zu- und abgeschaltet werden kann. Dabei wird eine Zuordnung der verschiedenen Widerstände zu den Bits der Konfigurationssequenz vorgenommen.

Die Ansteuerung durch die Steuereinheit 120 kann dann über verschiedene Anschlüsse, z.B. sieben Anschlüsse, erfolgen, wobei in diesem Fall dann jeweils zwei Gruppen aus drei Eingängen zur Konfiguration von Schieberegistern dienen und ein Eingang das PWM Signal zur Erzeugung der gewünschten Signalform des Ausgangsstromes aufnimmt. Alternativ können aber auch z.B. drei bis vier Anschlüsse für die Ansteuerung des Widerstandsnetzwerks, sechs Anschlüsse für die Auswahl der Wellenlänge (bzw. der anzusteuernden LED der Lichtquelle 110), ein Anschluss für das gepulste Anregungssignal (bzw. PWM Signal), zwei Anschlüsse zum Aktivieren von Stromtreiber und Widerstandsnetzwerk und ein Anschluss für Diagnosezwecke, z.B. zur Funktionsfähigkeit der Lichtquelle 110 und/oder Messeinheit 130, verwendet werden.

Die Konfiguration des variablen Widerstandes zur Stromstärkeneinstellung kann über zwei logisch in Reihe geschaltete Schieberegister (im Folgenden als SR2 abgekürzt) erfolgen, vorliegend mit Hilfe der Eingänge DATA_IN, CLK, CS, wobei CLK das Taktsignal, bei dessen steigender Flanke ein Datenübertrag stattfindet, CS das logisch invertierte Aktivierungssignal für den vollständigen, integrierten Schaltkreis einschließlich des Schieberegisters sowie DATA IN das einzulesende Datensignal ist. Für eine vollständige Konfiguration werden hier 8 bis 16 positive Taktflanken erzeugt, um die Schieberegisterkette vollständig neu zu füllen. Dabei steuert das erste übertragene Bit den Widerstand mit Index 16 und das letzte den Widerstand mit Index 1. Das andere Schieberegister (SR1) dient hier der Auswahl der anzusteuernden Lichtquelle 110 (bzw. der anzusteuernden LED der Lichtquelle 110) sowie zur Aktivierung/Deaktivierung des Stromtreiberbausteins STCS1A und der per internen Schieberegister konfigurierten Analogschalter. Hierbei werden die Eingänge DATA_IN, CLK, DISABLE zur Konfiguration eingesetzt, wobei diese sich von einem Schieberegister dahingehend unterscheidet, dass ein zusätzliches Ausgangsregister verbaut ist, welches unabhängig vom eigentlichen Datenregister (gespeist über den Takteingang SRCLK) aktiviert und deaktiviert werden kann. Außerdem ist die gezielte Übernahme von im Datenregister abgelegten Daten (über den gesonderten Takteingang RCLK) möglich. Dies ist vor allem für eine unterbrechungsfreie Aktualisierung der Ausgangsdaten wichtig.

Die Auswerteeinheit 140 gemäß **FIG. 1** ist so ausgebildet, dass sie basierend auf dem Messsignal ein Ausmaß der Flüssigkeitseinlagerung in der Harnblase 2 bestimmt und ein auf der Bestimmung basierendes Messungsergebnis ausgibt. Diese Bestimmung kann kontinuierlich bzw. fortlaufend erfolgen. Hierbei kann die Intensitätsamplitude einer Frequenzkomponente im Messsignal für die Bestimmung des Ausmaßes der Flüssigkeitseinlagerung ausschlaggebend sein. Das Ausmaß kann dann als Zahlen- oder Prozentwert von der vorab gemessenen Gesamtfüllmenge, oder auch als diskreter Wert, wie bspw. "leere Harnblase", "halbvolle Harnblase" oder "volle Harnblase" oder in einem gleitenden bzw. kontinuierlichen Spektrum von "leer" bis "voll", eingestuft werden. Dieses Ausmaß wird dann als Messungsergebnis ausgegeben. Somit basiert das Messungsergebnis auf der Bestimmung des Ausmaßes. Hierbei ist die Ausgabe bspw. das Bereitstellen des auszugebenden Messungsergebnisses, bzw. die Ausgabe des Messungsergebnisses für weitere Verarbeitung oder eine direkte Ausgabe des Messungsergebnisses als Hinweissignals. Das bestimmte Ausmaß kann ferner an der Auswerteeinheit angezeigt und/oder an eine externe Vorrichtung bevorzugt drahtlos, z.B. über eine Bluetooth-Schnittstelle oder dergleichen, übertragen werden.

Um Störfrequenzen durch andere Lichtquellen zu unterdrücken, kann die Messeinheit 130 oder die Auswerteeinheit 140 ferner so ausgebildet sein, dass sie andere Frequenzen um eine voreingestellte Frequenz aus dem Messsignal herausfiltert, hier bevorzugt in einem Frequenzfenster von 100 Hz - 10 kHz. Bei dieser voreingestellten Frequenz handelt es sich um die Trägerfrequenz von z.B. 500 Hz bis 5 kHz, wie vorstehend bereits erwähnt. Anschließend wird das gefilterte Messsignal zur Bestimmung des Ausmaßes der Flüssigkeitseinlagerung, wie vorstehend erläutert weiterverarbeitet. Eine derartige Signalnachbearbeitung wird z.B. mittels digitaler Filterung unter Nutzung eines schmalbandigen Bandpassfilters (bzw. dem Trägerfrequenzverfahren) durchgeführt, wobei das für die Weiterverarbeitung zu verwendende Messsignal herausgefiltert wird und Signalkomponenten mit höherer oder tieferer Frequenz unterdrückt werden. Der Bandpassfilter kann durch Software implementiert werden, z.B. mittels Korrelationsmethoden. Auch möglich sind FIR- (Finite Impulse Response) oder IIR (Infinite Impulse Response) - Filter mit frei konfigurierbarem Frequenzgang. Vorliegend kann vorteilhaft das Frequenzfenster von 100 Hz - 10 kHz verwendet werden; die Bandbreite beträgt dann 9.9 kHz. Dadurch können Fehler durch Störfrequenzen reduziert oder ganz unterdrückt werden, was wiederum die Messgenauigkeit bei der Bestimmung des Ausmaßes der Flüssigkeitseinlagerung erhöht. Durch Anpassung an die in der Messeinheit 130 eingesetzte Fotodiode und einer Transimpedanz-Verstärkerschaltung (wie z.B. in **Fig. 6** gezeigt) wird die voreingestellte Frequenz bestimmt. In der in **Fig. 6** gezeigten Transimpedanz-Verstärkerschaltung, kann z.B. ein Fotostrom $I_F$ der Fotodiode/n der Messeinheit 130 mit einem Widerstand $R_g$ im Gegenkopplungspfad in eine verstärkte Ausgangsspannung $U_a$ bzw. ein verstärktes Messsignal verstärkt werden. Eine derartige Signalnachbereitung kann aber sinnvoll nur dann erreicht werden, d. h. in einem vergleichsweise kurzen Zeitraum zur Bestimmung der Flüssigkeitseinlagerung, wenn die Taktung des Anregungssignals vergleichsweise hoch ist, insbesondere in einem Bereich von 500 bis 5 kHz, damit in diesem kurzen Zeitraum eine ausreichende Anzahl von Messwerten zur Verfügung steht.

Zur Anpassung des Anregungssignals an den Benutzer kann die Steuereinheit 120 ferner so ausgebildet sein, dass sie vor der (eigentlichen) Messung der Flüssigkeitseinlagerung eine Probemessung ausführt, bspw. durch einen oder mehrere Testpulse oder Test-Anregungssignale. Wenn diese Probemessung ergibt, dass die erfasste Lichtintensität, insbesondere bei der erläuterten voreingestellten Frequenz, unterhalb eines ersten vorbestimmten Schwellenwerts liegt, erhöht die Steuereinheit 120 die Stromstärke des an den Benutzer angepassten Anregungssignals. Eine Erhöhung der Stromstärke des Anregungssignals bewirkt vorliegend eine Erhöhung der Intensität des von der Lichtquelle ausgestrahlten Lichts. Es kann also von der Steuereinheit 120 festgestellt werden, dass die Anregung der Lichtquelle 110 zu gering ist und die Intensität des erfassen Lichts L2 oder der Signal-Rausch-Abstand zur optimalen Messung des erfassten Lichts L2 nicht ausreichend hoch ist. Durch Erhöhung der Amplitude, also der Stromstärke des Anregungssignals wird somit die Intensität des in den Benutzer eingestrahlten Lichts L1 erhöht und somit der Signal-Rausch-Abstand verbessert. Der erste vorbestimmte Schwellenwert kann demnach ein für die Messung erforderlicher Mindest-Signal-Rausch-Abstand sein oder ein absichtlich höher angesetzter Wert eines Signal-Rausch-Abstandes, um die Empfindlichkeit der Messvorrichtung 100 (bspw. durch Verschiebung/Verrutschen der Messvorrichtung beim Tragen am Körper des Benutzers) zu reduzieren. Die Messeinrichtung wird somit bevorzugt auf den Benutzer kalibriert und entsprechend individualisiert.

Alternativ oder zusätzlich kann die Steuereinheit 120 so ausgebildet sein, dass sie vor der Messung der Flüssigkeitseinlagerung eine Probemessung ausführt und, wenn die Probemessung ergibt, dass die erfasste Lichtintensität oberhalb eines zweiten vorbestimmten Schwellenwerts liegt, eine Stromstärke des an den Benutzer angepassten Anregungssignals verringern. Der zweite vorbestimmte Schwellenwert kann dabei so gewählt werden, dass eine Sättigung der Fotodiode oder des Infrarot-Detektors vermieden wird. Zusätzlich, da Infrarotbeleuchtung wärmt, kann auch eine unnötige Erwärmung durch das in den Benutzer eingestrahlte Licht L1 vermieden werden.

Nach einer (ersten) Probemessung können zusätzlich eine oder mehrere weitere Probemessungen folgen, sodass eine Änderung der Stromstärke in kleinen Intervallen es ermöglicht, genau auf einen optimalen Wert für die Messung der Flüssigkeitseinlagerung zuzulaufen. Parameter für die erste Probemessung, d.h. Parameter für das Anregungssignal eines ersten Testpulses, können bevorzugt aufgrund individueller Faktoren des Benutzers gewählt werden, wie z.B. Alter, Geschlecht, Gewicht oder auch Information bzgl. eines maximalen Blasenvolumens des Benutzers. Darüber können Parameter von vorhergehenden Messungen an dem Benutzer verwendet werden. Dies kann die Dauer des Probemessverfahrens verringern.

Um den Einfluss externer Umstände, die bspw. durch eine Verschiebung oder Schweiß ausgelöst werden, zu unterdrücken, kann die Steuereinheit 120 in einer weiteren Ausführungsform so ausgebildet sein, dass sie während der Messung der Flüssigkeitseinlagerung fortlaufend feststellt, ob die erfasste Lichtintensität oberhalb eines dritten vorbestimmten Schwellenwerts oder unterhalb eines vierten vorbestimmten Schwellenwerts liegt, und eine Stromstärke des an den Benutzer angepassten Anregungssignals entsprechend korrigieren. Der Vergleich mit dem dritten oder vierten vorbestimmten Schwellenwert kann fortlaufend durchgeführt werden, z.B. in vorgegebenen Zeitintervallen, um beispielweise ein nachträgliches Verschieben/Verrutschen der Messvorrichtung zu detektieren. Anhand dieser weiteren zwei Schwellenwerte wird somit ein optimaler Betriebsbereich definiert. Wenn dann bspw. der Signal-Rausch-Abstand außerhalb des für den optimalen Betrieb definierten Bereiches fällt, kann das Anregungssignal entsprechend korrigiert werden. Wenn die erfasste Lichtintensität den definierten Bereich verlässt, kann auch ein Alarmsignal, bspw. durch einen Lautsprecher (nicht abgebildet) oder durch ein optisches Signal, ausgegeben werden. Ein entsprechendes Alarmsignal kann auch an eine (später erläuterte) externe Überwachungseinheit ausgegeben werden. Die optimale Position der Messvorrichtung relativ zur Harnblase kann somit gewährleistet werden. Dabei ist einerseits die absolute Position zu Beginn einer Messung entscheidend, gleichzeitig wird gewährleistet, dass keine Verschiebung tangential zur Oberfläche stattfindet, wobei dieses Problem gerade während einer Miktion nicht zu unterschätzen ist, da hier Kontraktionen der Blasenmuskulatur bis zur Oberfläche übertragen werden.

Um die Messung der Flüssigkeitseinlagerung zu verbessern, kann die Lichtquelle 110 ferner mindestens eine erste und eine zweite Leuchtdiode aufweisen, die ausgebildet sind, Licht in einem Wellenlängenbereich von 600 nm bis 1200 nm, bevorzugt zwischen 600 nm bis 1000 nm, insbesondere zwischen 830 nm und 950 nm, auszustrahlen. Vorzugsweise werden vier Leuchtdioden mit unterschiedlichen Wellenlängen, z.B. 660 nm, 830 nm, 940 nm und 950 nm oder z.B. 670 nm, 830 nm, 1020 nm und 1050 nm eingesetzt. Immerhin haben, wie **FIG. 4** zu entnehmen ist, verschiedene Wellenlängen unterschiedliche Extinktionseigenschaften bei verschiedenen Gewebearten. Bei niedrigen Wellenlängen unter bspw. 700 nm haben Wasser, Blut und Melanin abweichende Extinktionskoeffizienten, während sich bei Wellenlängen über 700 nm diese Extinktionskoeffizienten annähern. Hierbei nähert sich jedoch erst der Extinktionskoeffizient von Wasser an den von Blut an und laufen dann auf den Extinktionskoeffizienten von Melanin zu. Wenn nun das Anregungssignal die Leuchtdioden (mit verschiedenen Wellenlängen) der Lichtquelle 110 unabhängig voneinander steuert, können die individuellen Verhältnisse der verschiedenen Gewebearten aufgrund der verschiedenen Extinktionskoeffizienten festgestellt werden. Es wird also eine Infrarotspektroskopie mit einer geringen Anzahl an spektralen Abtastpunkten durchgeführt, welche aber gleichwohl einen Rückschluss auf die Zusammensetzung des durchleuchteten Gewebes zulässt. Somit kann die Genauigkeit der Messung der Flüssigkeitseinlagerung verbessert werden.

Ein Abstand zwischen der Lichtquelle 110 und der Messeinheit 130 kann sich bevorzugt in einem Bereich von 25 mm bis 45 mm, insbesondere 30 mm befinden. Somit sind Lichtquelle 110 und Messeinheit 130 ausreichend voneinander entfernt, so dass hauptsächlich von der Harnblase zurückgeworfenes Licht von der Messeinheit 130 empfangen wird. Dies erhöht den Signal-Rausch-Abstand und optimiert die Messgenauigkeit.

Um gezielt Licht in den Körper 1 einzustrahlen und zu empfangen, können, wie in **FIG. 3** gezeigt, die Lichtquelle 110 und Messeinheit 130 so zueinander angeordnet sind, dass ein Ausstrahlvektor V1 der Lichtquelle (d.h. in einer Richtung des eingestrahlten Lichts) und ein Empfangsvektor V2 der Messeinheit (d.h. in einer Richtung des zurückgeworfenen Lichts) auf einen Fokuspunkt F (oder Brennpunkt) im Körper 1 des Benutzers zulaufen. Dieser Fokuspunkt F (oder Brennpunkt) befindet sich bevorzugt in einem Bereich nahe der dem Bauch zugewandten Seite der Oberfläche der Harnblase. Somit kann die Messung gezielt auf die Harnblase 2 abgestimmt und das Ausmaß der Flüssigkeitseinlagerung gemessen werden. Hierfür können Lichtquelle 110 und Messeinheit 130 in die Messvorrichtung 100 so eingesetzt werden, dass sie in Richtung des Fokuspunktes F zueinanderstehen. Um z.B. bei einer starren Messvorrichtung 100 ein Drehen oder Ablenken der Lichtquelle 110 und Messeinheit 130 zu vermeiden, können diese in der Messvorrichtung 100 fixiert werden. Die Fixierung kann z.B. durch eine Fixierungsschraube, Klebstoff oder Vergießen mit Flüssigharz oder Silikon erfolgen.

Hierbei kann ein Fokuswinkel $\alpha$ (oder Brennwinkel) zwischen dem Ausstrahlvektor V1 und dem Empfangsvektor V2 einen Wert im Bereich zwischen 0° und 90°, insbesondere einen Wert zwischen 0° und 60° und bevorzugt einen Wert zwischen 0° und 50° aufweisen. Somit ergibt sich eine bevorzugte Geometrie, bei das von der Harnblase zurückgeworfene Licht optimal von der Messeinheit 130 gemessen werden kann und Streulicht direkt von der Lichtquelle 110 zur Messeinheit 130 unterdrückt werden kann.

Um an verschiedene Körper verschiedener Benutzer anpassbar zu sein, ist der Fokuswinkel $\alpha$ einstellbar. Dies kann z.B. dadurch erreicht werden, dass der gewünschte Fokuswinkel $\alpha$ durch Drehung der Lichtquelle 110 und/oder Messeinheit 120 erzielt wird. Bspw. können auch bei der oben erwähnten starren Messungsvorrichtung 100, die Lichtquelle 110 und/oder Messeinheit 120 in der Messungsvorrichtung 100 gedreht werden, indem sie in eine dafür vorgesehene drehbare Halterung in die Messungsvorrichtung 100 eingesetzt werden. Demnach können Lichtquelle 110 und/oder Messeinheit 120 manuell justiert werden, bspw. über eine Einstellschraube oder, falls die Halterung ein Einrasten in gewünschten oder vorbestimmten Winkeln ermöglicht, über direktes Verdrehen der Halterung. Andernfalls oder zusätzlich kann auch ein automatisches Justieren der Lichtquelle 110 und/oder Messeinheit 120 erfolgen, bspw. indem ein Motor oder Aktor die Halterung dreht, oder ein Mikrosystem wie MEMS (englisch: "Micro-Electro-Mechanical System") die Halterung der Dioden der Lichtquelle 110 und/oder Messvorrichtung 130 dreht.

Um den Tragekomfort zu erhöhen und/oder eine Bewegungseinschränkung des Benutzers so klein wie möglich zu gestalten, kann zumindest die Steuereinheit 120, die Messeinheit 130 und die Auswerteeinheit 140 auf einer flexiblen Platine befestigt sein. Ein starres Gehäuse der Messungsvorrichtung 100 kann hierbei entfallen. Hierfür kann die Elektronik bspw. in einem flexiblen Kunststoff wie Silikon vergossen werden. Die flexible Platine kann sich insbesondere an die lokale Geometrie der Körperoberfläche des Benutzers anpassen und damit eine flexible Krümmung annehmen. Alternativ können die Steuereinheit 120, die Messeinheit 130 und die Auswerteeinheit 140 aber auch individuell in der Messvorrichtung aufgenommen werden, z.B. auf individuellen flexiblen Platinen, oder die Messvorrichtung weist zwei flexible Platinen auf, wobei die Steuereinheit bspw. auf einer ersten flexiblen Platine befestigt ist und die Messeinheit 130 und die Auswerteeinheit 140 auf einer zweiten flexiblen Platine befestigt ist.

Da sich bei Nutzung einer flexiblen Platine die Position und/oder Ausrichtung der Lichtquelle 110 und der Messeinheit 130 beim Tragen der Messvorrichtung 100 ändern kann, insbesondere bei Bewegungen des Benutzers, kann die Messeinheit 130 ferner ausgebildet sein, eine Auslenkung der flexiblen Platine (ausgehend von einer Anfangsposition bzw. Ruheposition, bei der sich z.B. die Krümmung der flexiblen Platine ändert) bei der Bestimmung des Ausmaßes der Flüssigkeitseinlagerung in der Harnblase 2 zu berücksichtigen. Eine Veränderung der Krümmung der flexiblen Platine sowie der Veränderung der Krümmung, die zu einer Veränderung des Abstands zwischen Lichtquelle und Messeinheit und/oder zu einer Veränderung des oben beschriebenen Fokuswinkels führt, kann z.B. mittels eines oder mehrerer zusätzlicher Beschleunigungsmesser (Gyroskop oder dergleichen) festgestellt werden, der/ die auf der flexiblen Platine befestigt ist/ sind. Hierfür können Beschleunigungs- oder Verdrehungsinformationen der Beschleunigungsmesser für Abschnitte der flexiblen Platine von der Messeinheit 130 zusammengeführt werden, um somit Aufschluss über die relative Position der einzelnen Abschnitte und somit die Veränderung der Krümmung oder der Ablenkung der Platine und/oder der Anordnung von Messeinheit 130 zur Lichtquelle 110 zu erhalten. Mit Hilfe der hierfür verwendeten Sensoren können zudem Bewegungsartefakte, also Messwerte, welche aufgrund einer Bewegung des Messsystems am Messobjekt stark beeinflusst wurden, identifiziert und entfernt werden. Ferner können durch Datenaustausch zwischen Messeinheit 130 und Lichtquelle 110 oder anhand veränderter Sensormessdaten der flexiblen Platine Rückschlüsse auf eine Verbiegung der Platine und somit Position und Ausrichtung der Lichtquelle 110 im Verhältnis zur Messeinheit 130 gezogen werden. Anhand dieser Rückschlüsse kann z.B. ein neuer Fokuspunkt F und Fokuswinkel $\alpha$ berechnet werden und somit Änderungen in Messwerten korrigiert werden. Andernfalls oder zusätzlich kann abhängig von der Veränderung der Ausrichtung und Anordnung von Lichtquelle 110 und Messeinheit 130, unter Nutzung der vorstehend beschriebenen automatischen Justierung, der Fokuspunkt F und/oder der Fokuswinkel $\alpha$ beibehalten werden. Dadurch kann sichergestellt werden, dass die Messung der Flüssigkeitseinlagerung im Wesentlichen identischen Messungsvoraussetzungen ausgesetzt ist und somit eine zuverlässigere fortlaufende Messung erfolgen kann. Bewegungsbedingte Änderungen im Messsignal können somit entsprechend korrigiert werden und entsprechende Datensätze müssen nicht mehr verworfen werden. Dadurch kann die Genauigkeit der Messung der Flüssigkeitseinlagerung bspw. während einer Bewegung des Benutzers weiter verbessert werden.

Zum Informieren des Benutzers oder Dritter (z.B. Ärzte, Pfleger oder Familienmitglieder) kann nach einer weiteren Ausführungsform die Auswerteeinheit 140 ferner ausgebildet sein, ein Signal an eine Überwachungseinheit zu übertragen, wenn das Ausmaß der Flüssigkeitseinlagerung in der Harnblase 2 einen Schwellenwert übersteigt. Somit kann bspw. der Benutzer vor einer ungewollten Miktion oder einem vesikorenalen Reflux rechtzeitig gewarnt werden und vor Beginn der Miktion eine Toilette aufsuchen oder die Katheterisierung vornehmen. Es können auch Dritte eine Meldung erhalten, falls der Benutzer nicht eigenständig in der 35 Lage ist, entsprechende Maßnahmen zu ergreifen, um vor Beginn der Miktion eine Toilette aufzusuchen. Die Überwachungseinheit kann hierbei eine mit der Auswerteeinheit in Verbindung stehende Vorrichtung sein, die den Benutzer durch Audio-, Video- und/oder Haptik-Signale informiert. Sie könnte hierfür mit einem Lautsprecher, einer Anzeige und/oder Vibrationseinheit ausgestattet sein. Die Überwachungseinheit ist hier bevorzugt eine tragbare (Wearable) Vorrichtung, z.B. ein Smartphone, eine Uhr, ein Armband oder dergleichen. Die Überwachungseinheit kann aber auch eine zentrale Überwachungseinheit, z.B. ein zentraler Computer in einer Überwachungseinrichtung wie einem Krankenhaus oder Pflegeheim sein, um bspw. über ein Netzwerk Dritte zu informieren.

Alternativ oder zusätzlich kann die Auswerteeinheit 140 ferner ausgebildet sein, ein Signal an die Überwachungseinheit zu übertragen, wenn erwartet wird, dass das Ausmaß der Flüssigkeitseinlagerung in der Harnblase 2 einen Schwellenwert in einem Zeitraum übersteigen wird. Bei dem Zeitraum kann es sich um einen vorbestimmten oder erwarteten Zeitraum handeln. Dieser vorbestimmte oder erwartete Zeitraum berücksichtigt, wie schnell sich die Harnblase füllt und damit eine Miktion zu erwarten ist. Somit kann der Benutzer und/oder das Betreuungspersonal rechtzeitig entsprechende Vorbereitungen vor Anbahnung einer Miktion treffen. Zusätzlich kann auch anhand des vorbestimmten oder erwarteten Schwellenwert auch das Überfüllen der Harnblase 2 festgestellt werden, um somit den Benutzer vor Gesundheitsschäden zu warnen. Anhand der erhaltenen Messergebnisse kann somit rechtzeitig in einem Zeitraum vor dem kalkulierten Zeitpunkt eines drohenden Urinverlusts oder des vesikorenalen Refluxes ein Alarmsignal oder dergleichen an den Betroffenen oder das Betreuungspersonal ausgegeben werden, bspw. an eine externe tragbare Vorrichtung (Smartphone, Uhr, Armband oder dergleichen) oder an zentrale Überwachungseinheit des Pflegepersonals, so dass der Benutzer dann rechtzeitig vor einer ungewollten Miktion oder bei unbewusstem Überfüllen der Harnblase eine Toilette aufsuchen kann. Der vorbestimmte bzw. erwartete Zeitraum ist einstellbar und kann somit individuell angepasst werden. Entsprechende Alarmsignale bzw. alarmierende Benachrichtigungen können auch mehrmals in einstellbaren Abständen und ggf. mit sukzessiv höherer Intensität (z.B. bei einem Ton-Signal) ausgegeben werden, sodass eine Erinnerungsfunktion implementiert werden kann. Mit dieser Vorgehensweise können ansonsten notwendige Hilfsmittel eingespart und die Lebensqualität und Gesundheit des Betroffenen durch die zurückerlangte Kontrolle über den Harnabgang deutlich erhöht werden.

Zur weiteren Verbesserung des Tragekomforts und/oder Reduzierung der Bewegungseinschränkung des Benutzers, kann die Messvorrichtung 100 ferner derart dimensioniert sein, dass sie am Körper 2 fixiert (z.B. mittels Pflaster oder dergleichen), in Textilien des Benutzers eingeschoben oder in einer für die Messvorrichtung 100 vorgesehenen Tasche getragen werden kann. Insbesondere kann die Messvorrichtung mit einem Clip am Gürtel, Gurt, Kleber oder Pflaster am Körper 2 des Benutzers fixiert werden, vorzugsweise etwa 2 cm oberhalb des Schambeins. Alternativ kann die Messvorrichtung 100 bspw. zwischen Hosenbund und Haut eingeschoben werden, wobei der Hosenbund als Halterung dient. Durch Stutzen oder Klammern kann zusätzlicher Halt am Hosenbund gewährleistet werden, sodass die Messvorrichtung 100 nicht verrutscht oder aus ihrer Halterung fällt. Alternativ kann die Messvorrichtung auch in die Textilien wie Unterwäsche oder absorbierende Hilfsmittel wie Windeln eingeschoben werden und/oder vernäht werden. Eine für die Messvorrichtung 100 vorgesehene Tasche ist eine Tasche des Textils, die es der Messvorrichtung 100 ermöglicht, die Messung der Flüssigkeitseinlagerung durchzuführen, wenn die Messvorrichtung 100 in die Tasche eingeschoben wird. Löcher in der Innenseite der Tasche oder Nutzung von infrarotdurchlässigen Textilien zur Herstellung der Tasche würden die Messung ermöglichen. Derartige Maßnahmen bewirken einen konstanten Anpressdruck der Messvorrichtung an die Haut des Benutzers und gewährleisten somit eine gleichbleibende Ein- und Auskoppelung des Anregungslichtsignals.

Die Auswerteeinheit 140 kann bevorzugt ferner ausgebildet sein, eine Autokorrelation oder eine Kreuzkorrelation mit einem vorbestimmten Signal zu verwenden, um eine Signal- und Rauschleistung zu berechnen und daraus eine Signalqualität zu bestimmen, auf Basis dessen die Steuereinheit 120 eine Helligkeit der Lichtquelle 110 anpasst. Bei der Signalqualität handelt es sich bspw. um ein Signal-zu-Rauschverhältnis (SNR), d.h. um das Verhältnis aus Signalleistung (bzw. Nutzsignalleistung) und Rauschleistung, wodurch eine Unsicherheit des Messignals, insbesondere eine Unsicherheit der Signalleistung abgeschätzt werden kann.

**Fig. 9** zeigt dazu als Beispiel ein detektiertes Signal nach Verstärkung und Bereinigung des Mittelwerts (durchzogene Linie; mit Signalamplitude in mV) und das zugrundeliegende Anregungssignal (gepunktet, entsprechend **Fig. 8**) für zwei beispielhafte Messungen. Ein Verfahren zur Bestimmung der Signalleistung und der Rauschleistung beruht bevorzugt auf der Extraktion eines periodischen Signalanteils aus einem verrauschten Signal. Die Frequenz und die Kurvenform des periodischen Signalanteils sind bekannt, da sie durch den IR-Emitter (Lichtquelle 110) vorgegeben werden. Zusammenfassend kann das detektierte Signal y folgendermaßen dargestellt werden:

$$y(t) = s(t) + n(t) + c \qquad\qquad (1.1)$$

Hierbei ist $s(t)$ das Nutzsignal, $n(t)$ der zufällige Rauschanteil und c eine unbekannte, konstante Abweichung durch verschiedene Störeinflüsse, wie z.B. die Körperwärme oder Komponenten des Tageslichts.

Mit Hilfe einer Autokorrelation kann der periodische Anteil des empfangenen Signals bestimmt werden. Dafür wird bevorzugt sichergestellt, dass das detektierte Signal mittelwertfrei ist. Dies kann dadurch erreicht werden, dass der Mittelwert des gemessenen Signals berechnet und anschließend von jedem Messpunkt des Signals subtrahiert wird. Die Berechnung der vollständigen Autokorrelation eines Signals kann aufwändig sein. Bei der vorliegenden nicht-invasiven Messvorrichtung mit eingeschränkten Ressourcen ist es daher notwendig, den Rechenaufwand zu reduzieren, während gleichzeitig die notwendigen Informationen erhalten bleiben. Dazu können folgende Größen bestimmt werden: Gesamtleistung: Die gesamte Signalleistung des Signals y(t) = s(t) + n(t) + c entspricht der Autokorrelation $\Phi_{yy}$ bei der

Verschiebung $\tau = 0$, also $P_{ges,y} = \Phi_{yy}(\tau = 0)$.

Signalleistung: Die Leistung des Nutzsignals entspricht der Amplitude des periodischen Signalanteils der Autokorrelation. Es ist zu beachten, dass aufgrund der zeitlichen Fensterung des Messsignals (endliche Messzeit T = 10 ms) die Amplitude der Autokorrelation mit wachsender Verschiebung abklingt.

Rauschleistung: Die Rauschleistung ergibt sich als Differenz der Gesamtleistung und der Signalleistung, ist also durch die beiden vorherigen Punkte definiert.

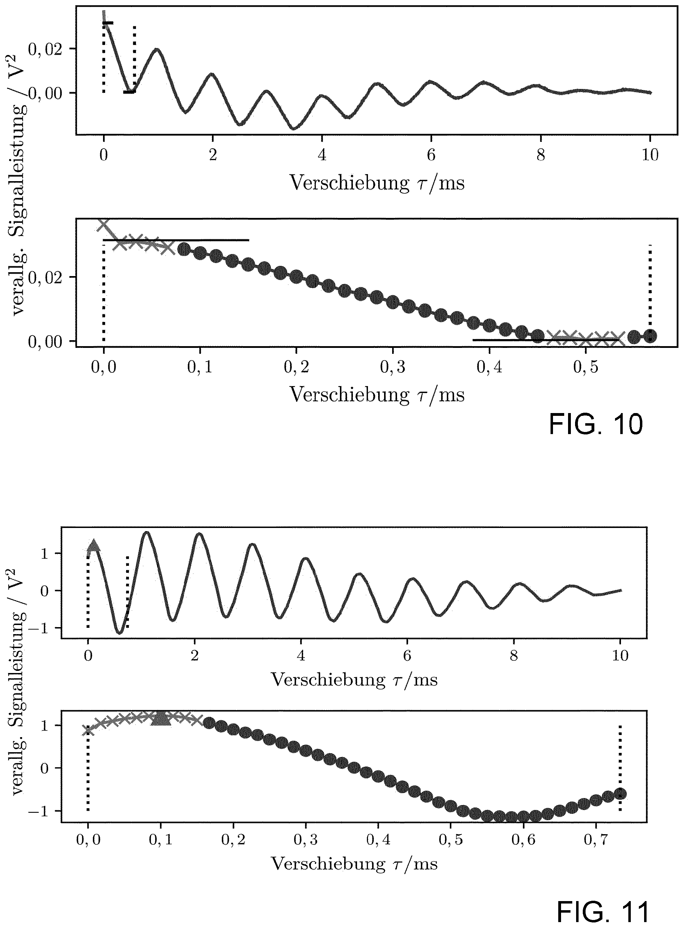

Eine vorteilhafte Berechnung der Amplitude des periodischen Anteils der Autokorrelation entscheidet über die Effizienz der gesamten Signalauswertung. **Fig. 10** zeigt eine Autokorrelation eines mittelwertfreien Signals (oberes Signal in **Fig. 9**) in verschieden großen Zeitausschnitten (oben: Verschiebung $\tau$ zwischen 0 und 10 ms; unten: Verschiebung $\tau$ zwischen 0 und 0.5 ms). Für eine effiziente Berechnung werden bevorzugt die in **Fig. 10** durch ($\times$) markierten Punkte verwendet. Die Auswahl dieser Punkte geschieht relativ zur Periodendauer des emittierten Signals, insbesondere im Bereich eins Maximums und/oder eines Minimums der Autokorrelation. Da also vorab bekannt ist, wo Minima und Maxima des periodischen Signalanteils der Autokorrelation liegen müssen, hat dies zur Folge, dass die Autokorrelation nicht über alle Verschiebungen $\tau$ berechnet werden muss, sondern nur ein Bruchteil aller denkbaren Verschiebungen. Der Fachmann erkennt, dass die Autokorrelation unempfindlich gegenüber einer Phasenverschiebung gegenüber dem Anregungssignal ist.

Alternativ oder zusätzlich kann eine Kreuzkorrelation verwendet werden, hier bevorzugt eine Kreuzkorrelation des empfangenen Signals mit einem normierten und mittelwertfreien Anregungssignal, z.B. einem Rechtecksignal. **Fig. 11** zeigt eine Kreuzkorrelation eines mittelwertfreien Signals (oberes Signal in **Fig. 9**) für verschiedene zeitliche Verschiebungen in verschieden großen Zeitausschnitten (oben: Verschiebung $\tau$ zwischen 0 und 10 ms; unten: Verschiebung $\tau$ zwischen 0 und 0.7 ms). Der Phasenversatz wurde hier noch nicht ausgeglichen. Die Markierung ($\times$) kennzeichnet den Suchbereich für das lokale Maximum der Kreuzkorrelation. Ist der Phasenversatz einmal bestimmt, genügt es, die Kreuzkorrelation für einen Punkt (Maximum der Kreuzkorrelation, gekennzeichnet durch Markierung ($\Delta$) in **Fig. 11**) zu berechnen. Die Entfernung des Mittelwerts vom detektierten (abgetasteten) Signal bzw. Messsignal entfällt in diesem Fall, da der ggf. vorhandene Mittelwert als konstanter Offset mit dem mittelwertfreien korrelierten Signal nicht korreliert und somit keinen Beitrag zur Kreuzkorrelation leistet. Ebenso korreliert das normierte Testsignal nicht mit dem zufälligen Rauschanteil, womit auch dieser Anteil aus der Kreuzkorrelation entfällt. Übrig bleibt nach Gleichung (1.5) damit nur die Korrelation aus dem Nutzsignalanteil und dem bekannten Testsignal.

$$\Phi_{yx} = \int_0^T y(t) \cdot x(t - \tau)\, d\tau = \tag{1.2}$$

$$= \int_0^T [s(t) + n(t) + c] \cdot sq(t - \tau)\, d\tau = \tag{1.3}$$

$$= \int_0^T [s(t) \cdot sq(t - \tau)]\, d\tau + \underbrace{\int_0^T [n(t) \cdot sq(t - \tau)]\, d\tau}_{\approx 0} + \underbrace{\int_0^T [c \cdot sq(t - \tau)]\, d\tau}_{\approx 0} = \tag{1.4}$$

$$= \int_0^T [s(t) \cdot sq(t - \tau)]\, d\tau \tag{1.5}$$

Hierbei ist das detektierte Signal $y(t)$, das rechteckförmige und mittelwertfreie Test- oder Referenzsignal $x(t) = sq(t)$, das Nutzsignal $s(t)$, der Rauschanteil $n(t)$ und der konstante Offsetfehler $c$. Zu beachten ist, dass die Kreuzkorrelation im Gegensatz zur Autokorrelation empfindlich auf einen Phasenversatz zwischen Anregungssignal und detektiertem Signal reagiert. Dieser ist in der beschriebenen Messvorrichtung aufgrund der vergleichsweise trägen Reaktion des Detektors vorhanden. Vor der Nutzung der Kreuzkorrelation muss diese Phasenverschiebung bestimmt werden. Dies kann über eine vollständige Berechnung der ersten erwarteten Periode ($\tau \in [0, T/10]$) der Kreuzkorrelation und anschließender Suche des lokalen Maximums im in **Fig. 11** gekennzeichneten Bereich geschehen. Anschließend muss pro Messung nur noch für eine Verschiebung $\tau$ die Kreuzkorrelation berechnet werden. Anschließend können die gesuchten Größen Signal- und Rauschleistung effizient berechnet werden:

Gesamtleistung: Die gesamte Leistung des Messsignals $y(t)$ kann über die Summe der quadrierten Signalwerte bestimmt werden. Dies entspricht der Autokorrelation $\Phi_{yy}$ bei $\tau = 0$.

Signalleistung: Die Signalleistung entspricht der Kreuzkorrelation des detektierten Signals mit dem in Phase gebrachten Referenzsignal bei einer Verschiebung von $\tau = 0$.

Rauschleistung: Die Rauschleistung ergibt sich als Differenz der Gesamtleistung und der Signalleistung.

Ansprechend auf die bestimmte Signalqualität, bspw. gegeben durch das Signal-zu-Rauschverhältnis (SNR), d.h. um das Verhältnis aus berechneter Signalleistung (bzw. Nutzsignalleistung) und Rauschleistung, kann die Steuereinheit 120 eine Helligkeit der Lichtquelle 110 geeignet anpassen. Dabei kann vorzugsweise folgender Mechanismus verwendet werden.

Wenn das SNR unterhalb eines geeignet zu wählenden ersten SNR-Schwellenwerts liegt, d.h. ein relativ großer Rauschanteil vorhanden ist und das Messsignal mit einer vergleichsweise großen Unsicherheit belegt ist, kann zuerst die Signalleistung (Helligkeit der Lichtquelle) in einem gewissen Rahmen (begrenzt durch Belastbarkeit der Lichtquellen/IR-LEDs, der Erwärmung des Gewebes und der Kapazität des Energiespeichers/ Akkus) erhöht werden. Wenn dies nicht zum gewünschten Erfolg führt (weil entweder nur ein zu geringer Signalanteil an der Messeinheit 130 ankommt, oder weil die Rauschleistung zu groß ist), sollte die Messung pausiert werden, da die Messwerte wahrscheinlich fehlerbehaftet sind. Während der Pause werden vereinzelt (in festen Zeitabständen, die deutlich größer sind als die Zeitabstände zwischen regulären Messungen, die z.B. im Bereich von 5 Sekunden bis 5 Minuten liegen, Versuche (Testmessungen) unternommen, die Messung wieder zu beginnen. Wenn bei einem solchen Versuch wieder ein höheres SNR (d.h. oberhalb des ersten SNR-Schwellenwerts) erzielt wird, kann die Pause beendet werden.

Wenn das SNR andererseits oberhalb eines geeignet zu wählenden zweiten SNR-Schwellenwert liegt, d.h. ein relativ geringer Rauschanteil vorhanden ist und das Messsignal mit einer vergleichsweise geringen Unsicherheit belegt ist, kann die Signalleistung (Helligkeit der Lichtquelle) verringert werden und damit vorteilhaft der Energieverbrauch gesenkt werden, wenn mit weniger Helligkeit in den Körper einstrahlt wird. Mit anderen Worten kann hier stückweise die Signalleistung reduziert werden, bis das SNR den zweiten SNR-Schwellenwert unterschreitet. Der zweite SNR-Schwellenwert kann insbesondere empirisch so gewählt werden, dass sich ein Kompromiss aus Energieverbrauch und SNR ergibt.

Wenn andererseits das SNR in einem Bereich zwischen dem ersten und dem zweiten SNR-Schwellenwert liegt, muss die Helligkeit der Lichtquelle nicht angepasst werden.

## Patentansprüche

1. Eine nicht-invasive Messvorrichtung (100) zur Messung einer Flüssigkeitseinlagerung in der Harnblase (2) eines Benutzers, mit:

    einer Lichtquelle (110), die ausgebildet ist, auf dem Körper (1) des Benutzers anzuliegen und Licht (L1) in den Benutzer einzustrahlen;
    einer Steuereinheit (120), die ausgebildet ist, ein an den Benutzer angepasstes Anregungssignal, das die Lichtausstrahlung der Lichtquelle (110) steuert, an die Lichtquelle (110) auszugeben;
    einer Messeinheit (130), die ausgebildet ist, auf dem Körper (1) des Benutzers anzuliegen, das von der Lichtquelle (110) ausgestrahlte und von der Harnblase (2) reflektierte Licht (L2) zu erfassen und ein dem erfassten Licht entsprechendes Messsignal auszugeben; und
    einer Auswerteeinheit (140), die ausgebildet ist, basierend auf dem Messsignal, ein Ausmaß der Flüssigkeitseinlagerung in der Harnblase (2) zu bestimmen und ein auf der Bestimmung basierendes Messungsergebnis auszugeben,
    **dadurch gekennzeichnet, dass**
    die Lichtquelle (110) und Messeinheit (130) so zueinander angeordnet sind, dass ein Ausstrahlvektor (V1) der Lichtquelle und ein Empfangsvektor (V2) der Messeinheit auf einen Fokuspunkt (F) im Körper (1) des Benutzers zulaufen; und
    ein Fokuswinkel ($\alpha$) zwischen dem Ausstrahlvektor (V1) und dem Empfangsvektor (V2) einen Wert im Bereich zwischen 0° und 50° aufweist, wobei der Fokuswinkel ($\alpha$) einstellbar ist.

2. Die Messvorrichtung (100) nach Anspruch 1, wobei
    die Messeinheit (130) oder die Auswerteeinheit (140) ausgebildet ist, andere Frequenzen um eine voreingestellte Frequenz aus dem Messsignal herauszufiltern.

3. Die Messvorrichtung (100) nach Anspruch 1 oder 2, wobei
    die Steuereinheit (120) ausgebildet ist, vor der Messung der Flüssigkeitseinlagerung eine Probemessung auszuführen und, wenn die Probemessung ergibt, dass die erfasste Lichtintensität unterhalb eines ersten vorbestimmten Schwellenwerts liegt, eine Stromstärke des an den Benutzer angepassten Anregungssignals zu erhöhen.

4. Die Messvorrichtung (100) nach einem der Ansprüche 1 bis 3, wobei
    die Steuereinheit (120) ausgebildet ist, vor der Messung der Flüssigkeitseinlagerung eine Probemessung auszuführen und, wenn die Probemessung ergibt, dass die erfasste Lichtintensität oberhalb eines zweiten vorbestimmten Schwellenwerts liegt, eine Stromstärke des an den Benutzer angepassten Anregungssignals zu verringern.

**5.** Die Messvorrichtung (100) nach einem der Ansprüche 1 bis 4, wobei

die Steuereinheit (120) ausgebildet ist, während der Messung der Flüssigkeitseinlagerung fortlaufend festzustellen, ob die erfasste Lichtintensität oberhalb eines dritten vorbestimmten Schwellenwerts oder unterhalb eines vierten vorbestimmten Schwellenwerts liegt, und
eine Stromstärke des an den Benutzer angepassten Anregungssignals entsprechend zu korrigieren.

**6.** Die Messvorrichtung (100) nach einem der Ansprüche 1 bis 5, wobei

die Lichtquelle (110) mindestens eine erste und eine zweite Leuchtdiode umfasst, die ausgebildet sind, ein Licht in einem Wellenlängenbereich von 600 nm bis 1200 nm auszustrahlen; und
das Anregungssignal die Leuchtdioden der Lichtquelle (110) unabhängig voneinander steuert.

**7.** Die Messvorrichtung (100) nach einem der Ansprüche 1 bis 6, wobei
ein Abstand zwischen der Lichtquelle (110) und der Messeinheit (130) sich im Bereich von 25 mm bis 45 mm, insbesondere 30 mm befindet.

**8.** Die Messvorrichtung (100) nach einem der Ansprüche 1 bis 7, wobei
der Fokuspunkt (F) sich im Bereich der Harnblase (2) befindet.

**9.** Die Messvorrichtung (100) nach einem der Ansprüche 1 bis 8, wobei
zumindest die Steuereinheit (120), die Messeinheit (130) und die Auswerteeinheit (140) auf einer flexiblen Platine befestigt sind.

**10.** Die Messvorrichtung (100) nach Anspruch 9, wobei
die Messeinheit (130) ferner ausgebildet ist, eine Auslenkung der flexiblen Platine bei der Bestimmung des Ausmaßes der Flüssigkeitseinlagerung in der Harnblase (2) zu berücksichtigen.

**11.** Die Messvorrichtung (100) nach einem der Ansprüche 1 bis 10, wobei
die Auswerteeinheit (140) ferner ausgebildet ist, ein Signal basierend auf dem Messungsergebnis an eine zentrale Überwachungseinheit zu übertragen, wenn das Ausmaß der Flüssigkeitseinlagerung in der Harnblase (2) einen vorbestimmten Schwellenwert übersteigt.

**12.** Die Messvorrichtung (100) nach einem der Ansprüche 1 bis 10, wobei
die Auswerteeinheit (140) ferner ausgebildet ist, ein Signal basierend auf dem Messungsergebnis an eine zentrale Überwachungseinheit zu übertragen, wenn das Ausmaß der Flüssigkeitseinlagerung in der Harnblase (2) einen vorbestimmten Schwellenwert in einer vorbestimmten Zeit übersteigen wird.

**13.** Die Messvorrichtung (100) nach einem der Ansprüche 1 bis 12, wobei
die Messvorrichtung (100) derart dimensioniert ist, dass sie in Textilien des Benutzers eingeschoben werden kann.

**14.** Die Messvorrichtung (100) nach einem der Ansprüche 1 bis 13, wobei
die Auswerteeinheit (140) ferner ausgebildet ist, eine Autokorrelation und/oder eine Kreuzkorrelation mit einem vorbestimmten Signal zu verwenden, um eine Signal- und Rauschleistung zu berechnen und daraus eine Signalqualität zu bestimmen, auf Basis dessen die Steuereinheit (120) eine Helligkeit der Lichtquelle (110) anpasst.

**Claims**

**1.** A non-invasive measuring device (100) for measuring fluid retention in the urinary bladder (2) of a user, comprising:

a light source (110) adapted to rest against the body (1) of the user and to irradiate light (L1) into the user;
a control unit (120) adapted to output to the light source (110) an excitation signal matched to the user and controlling the light emission of the light source (110);
a measuring unit (130) which is designed to rest against the body (1) of the user, to detect the light (L2) emitted by the light source (110) and reflected by the urinary bladder (2), and to output a measurement signal corresponding to the detected light; and
an evaluation unit (140) adapted to determine an amount of fluid retention in the urinary bladder (2) based on the measurement signal and to output a measurement result based on the determination, **characterized in that** the light source (110) and measuring unit (130) are arranged with respect to each other such that an emission vector (V1) of the light source and a reception vector (V2) of the measuring unit converge to a focal point (F) in the body (1) of the user; and

a focus angle ($\alpha$) between the emitting vector (V1) and the receiving vector (V2) has a value in the range between 0° and 50°, wherein the focus angle ($\alpha$) is adjustable.

2. The measuring device (100) according to claim 1, wherein the measuring unit (130) or the evaluation unit (140) is adapted to filter out other frequencies around a preset frequency from the measurement signal.

3. The measuring device (100) according to claim 1 or 2, wherein the control unit (120) is adapted to perform a sample measurement prior to measuring the fluid retention and, if the sample measurement indicates that the detected light intensity rests against below a first predetermined threshold, to increase a current intensity of the excitation signal matched to the user.

4. The measuring device (100) according to any one of claims 1 to 3, wherein the control unit (120) is adapted to perform a sample measurement prior to measuring the fluid retention and, if the sample measurement indicates that the detected light intensity is above a second predetermined threshold, to decrease a current intensity of the excitation signal matched to the user.

5. The measuring device (100) according to any one of claims 1 to 4, wherein

the control unit (120) is adapted to continuously determine, during the measurement of the fluid retention, whether the detected light intensity is above a third predetermined threshold or below a fourth predetermined threshold; and

correcting a current intensity of the user matched excitation signal accordingly.

6. The measuring device (100) according to any one of claims 1 to 5, wherein.

the light source (110) comprises at least a first and a second light emitting diode adapted to emit a light in a wavelength range of 600 nm to 1200 nm; and

the excitation signal independently controls the light diodes of the light source (110).

7. The measuring device (100) according to any one of claims 1 to 6, wherein. a distance between the light source (110) and the measuring unit (130) is in the range of 25 mm to 45 mm, in particular 30 mm.

8. The measuring device (100) according to any one of claims 1 to 7, wherein the focal point (F) is located in the region of the urinary bladder (2).

9. The measuring device (100) according to any one of claims 1 to 8, wherein at least the control unit (120), the measuring unit (130) and the evaluation unit (140) are mounted on a flexible circuit board.

10. The measuring device (100) according to claim 9, wherein the measuring unit (130) is further adapted to take into account a deflection of the flexible circuit board when determining the extent of fluid retention in the urinary bladder (2).

11. The measuring device (100) according to any one of claims 1 to 10, wherein the evaluation unit (140) is further adapted to transmit a signal based on the measurement result to a central monitoring unit when the extent of fluid retention in the urinary bladder (2) exceeds a predetermined threshold.

12. The measuring device (100) according to any one of claims 1 to 10, wherein the evaluation unit (140) is further adapted to transmit a signal based on the measurement result to a central monitoring unit when the extent of fluid retention in the urinary bladder (2) will exceed a predetermined threshold in a predetermined time.

**13.** The measuring device (100) according to any one of claims 1 to 12, wherein
the measuring device (100) is dimensioned such that it can be inserted into textiles of the user.

**14.** The measuring device (100) according to any one of claims 1 to 13, wherein
the evaluation unit (140) is further configured to use autocorrelation and/or cross-correlation with a predetermined signal to calculate a signal and noise power and determine therefrom a signal quality, based on which the control unit (120) matches a brightness of the light source (110).

**Revendications**

**1.** Dispositif de mesure (100) non invasif destiné à la mesure d'une rétention de liquide dans la vessie (2) d'un utilisateur, avec :

une source lumineuse (110), qui est conçue pour être appliquée sur le corps (1) de l'utilisateur et projeter de la lumière (L1) dans l'utilisateur ;
une unité de commande (120), qui est conçue pour émettre un signal d'excitation adapté à l'utilisateur, qui commande le rayonnement lumineux de la source lumineuse (110), à la source lumineuse (110) ;
une unité de mesure qui est conçue pour être appliquée sur le corps (1) de l'utilisateur afin de détecter de la lumière (L2) diffusée par la source lumineuse (110) et réfléchie par la vessie (2) et émettre un signal de mesure correspondant à la lumière détectée ; et
une unité d'évaluation (140), qui est conçue pour, en s'appuyant sur le signal de mesure, déterminer un degré de la rétention de liquide dans la vessie (2) et émettre un résultat de mesure s'appuyant sur la détermination, **caractérisé en ce que**
la source lumineuse (110) et l'unité de mesure (130) sont disposées l'une par rapport à l'autre de telle manière qu'un vecteur d'émission (V1) de la source lumineuse et un vecteur de réception (V2) de l'unité de mesure se dirigent vers un point focal (F) dans le corps (1) de l'utilisateur ; et
un angle de focalisation ($\alpha$) entre le vecteur d'émission (V1) et le vecteur de réception (V2) présente une valeur dans la plage comprise entre 0° et 50°, dans lequel l'angle de focalisation ($\alpha$) peut être réglé.

**2.** Dispositif de mesure (100) selon la revendication 1, dans lequel
l'unité de mesure (130) ou l'unité d'évaluation (140) est conçue pour filtrer d'autres fréquences à une fréquence prédéfinie du signal de mesure.

**3.** Dispositif de mesure (100) selon la revendication 1 ou 2, dans lequel
l'unité de commande (120) est conçue pour effectuer une mesure de l'échantillon avant la mesure de la rétention de liquide et, si la mesure de l'échantillon révèle que l'intensité lumineuse détectée est inférieure à un premier seuil prédéterminé, augmenter une intensité du courant du signal d'excitation adapté à l'utilisateur.

**4.** Dispositif de mesure (100) selon l'une quelconque des revendications 1 à 3, dans lequel
l'unité de commande (120) est conçue pour effectuer une mesure de l'échantillon avant la mesure de la rétention de liquide et, si la mesure de l'échantillon révèle que l'intensité lumineuse détectée est supérieure à un deuxième seuil prédéterminé, diminuer une intensité du courant du signal d'excitation adapté à l'utilisateur.

**5.** Dispositif de mesure (100) selon l'une quelconque des revendications 1 à 4, dans lequel

l'unité de commande (120) est conçue pour établir continuellement, pendant la mesure de la rétention de liquide, si l'intensité lumineuse détectée est supérieure à un troisième seuil prédéterminé ou inférieure à un quatrième seuil prédéterminé, et
corriger en conséquence une intensité du courant du signal d'excitation adapté à l'utilisateur.

**6.** Dispositif de mesure (100) selon l'une quelconque des revendications 1 à 5, dans lequel

la source lumineuse (110) comprend au moins une première et une seconde diode électroluminescente, qui sont conçues pour diffuser une lumière suivant une plage de longueurs d'onde allant de 600 nm à 1200 nm ; et
le signal d'excitation commande les diodes électroluminescentes de la source lumineuse (110) indépendamment les unes des autres.

**7.** Dispositif de mesure (100) selon l'une quelconque des revendications 1 à 6, dans lequel
une distance entre la source lumineuse (110) et l'unité de mesure (130) se situe dans la plage allant de 25 mm à 45 mm, en particulier 30 mm.

**8.** Dispositif de mesure (100) selon l'une quelconque des revendications 1 à 7, dans lequel
le point focal (F) se situe dans la zone de la vessie (2).

**9.** Dispositif de mesure (100) selon l'une quelconque des revendications 1 à 8, dans lequel
au moins l'unité de commande (120), l'unité de mesure (130) et l'unité d'évaluation (140) sont fixées à un circuit imprimé souple.

**10.** Dispositif de mesure (100) selon la revendication 9, dans lequel
l'unité de mesure (130) est conçue en outre pour prendre en compte une déviation du circuit imprimé souple lors de la détermination du degré de la rétention de liquide dans la vessie (2).

**11.** Dispositif de mesure (100) selon l'une quelconque des revendications 1 à 10, dans lequel
l'unité d'évaluation (140) est conçue en outre pour transmettre un signal s'appuyant sur le résultat de mesure à une unité de surveillance centrale, lorsque le degré de la rétention de liquide dans la vessie (2) dépasse un seuil prédéterminé.

**12.** Dispositif de mesure (100) selon l'une quelconque des revendications 1 à 10, dans lequel
l'unité d'évaluation (140) est conçue en outre pour transmettre un signal s'appuyant sur le résultat de mesure à une unité de surveillance centrale, lorsque le degré de la rétention de liquide dans la vessie (2) dépasse un seuil prédéterminé au cours d'une période prédéterminée.

**13.** Dispositif de mesure (100) selon l'une quelconque des revendications 1 à 12, dans lequel
le dispositif de mesure (100) est dimensionné de telle sorte qu'il peut être inséré dans les vêtements de l'utilisateur.

**14.** Dispositif de mesure (100) selon l'une quelconque des revendications 1 à 13, dans lequel
l'unité d'évaluation (140) est conçue en outre pour utiliser une autocorrélation et/ou une corrélation croisée avec un signal prédéterminé pour calculer une puissance de signal et de bruit et déterminer à partir de celles-ci une qualité de signal, sur la base de laquelle l'unité de commande (120) adapte une luminosité de la source lumineuse (110).

FIG. 1

FIG. 2

FIG. 3

FIG. 4

FIG. 5

FIG. 6

FIG. 7

FIG. 8

FIG. 9

FIG. 10

FIG. 11

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20060276712 A1

- WO 2018175100 A1

**In der Beschreibung aufgeführte Nicht-Patentliteratur**

- **GEHIRN. MOLAVI et al.** *Muskelparametern bei Sportlern oder Informationen*